# EUROPEAN PATENT APPLICATION

(11) **EP 4 437 993 A1**
(43) Date of publication of application: **02.10.2024**
(21) Application number: 23165415.3
(22) Date of filing: 30.03.2023
(51) Int. Cl.: A61B 34/20, A61B 34/30

(54) **DEVICE TRACKING AND MONITORING OUTSIDE IMAGING FIELD OF VIEW**

(71) Applicant: Koninklijke Philips N.V., 5656 AG Eindhoven (NL)
(72) Inventor: FOTOUHI, javad, Eindhoven (NL); BALICKI, Marcin A., Eindhoven (NL); PATRICIU, Alexandru, 5656AG Eindhoven (NL)
(74) Representative: Philips Intellectual Property & Standards

(57) **Abstract**

A system includes a memory, a processor, and a motor. Instructions from the memory cause the system to: receive images; receive encoding data corresponding to driving of an elongated device by the motor; determine positions of the elongated device; determine when at least a portion of the elongated device is outside of a field of view in the images or likely to leave the field of view in the images; determine whether to create extended image data to show positions of the elongated device outside of the field of view in the images; determine current locations, past locations and/or predicted locations of the elongated device outside of the field of view in the images based on pixel data in the images and the encoding data; and extend the images to show the locations of the elongated device outside of the field of view in the images in extended images.

## Description

### BACKGROUND

Robot assistance during endovascular procedures is used to improve clinical outcomes and care delivery under fluoroscopic guidance. Among the potential benefits of using robot assistance are increasing safety and reducing the total fluoroscopy acquisition during endovascular procedures performed under the fluoroscopic guidance.

Endovascular robots allow automatic or guided navigation of endovascular devices inside patient vasculature using motorized actuation units. Endovascular devices such as catheters and guidewires are navigated using endovascular robots during endovascular procedures under fluoroscopic guidance, implying continuous radiation. Nevertheless, it may happen that the guidewire tip leaves the field of view in the fluoroscopic imagery and becomes invisible. To reacquire the guidewire tip, additional X-ray images may be required and so additional radiation for the patient and/or the surgical personal would be needed.

### SUMMARY

According to an aspect of the present disclosure, a system for tracking an elongated device in an anatomy includes a memory, a processor, and a motor. The memory stores instructions. The processor executes the instructions. When executed by the processor, the instructions cause the system to: receive images; receive encoding data corresponding to driving of an elongated device by the motor; determine positions of the elongated device; determine when at least a portion of the elongated device is outside of a field of view in the images or likely to leave the field of view in the images; determine whether to create extended image data to show positions of the elongated device outside of the field of view in the images; determine at least one of current locations, past locations or predicted locations of the elongated device outside of the field of view in the images based on pixel data in the images and the encoding data; and extend the images to show the locations of the elongated device outside of the field of view in the images in extended images.

Further embodiments of this aspect of the present disclosure are also described based on claims 2 through 9, considered alone or in any combination.

According to another aspect of the present disclosure, a method for tracking an elongated device in an anatomy includes: receiving, at a system comprising a memory that stores instructions and a processor that executes the instructions, images of an elongated device; receiving encoding data corresponding to driving of the elongated device; determining positions of the elongated device; determining when at least a portion the elongated device is outside of a field of view in the images or likely to leave the field of view in the images; determining whether to create extended image data to show positions of the elongated device outside of the field of view in the images; determining current or predicted locations of the elongated device outside of the field of view in the images based on the pixel data and the encoding data; and extending the images to show the locations of the elongated device outside of the field of view in the images in extended images.

Further embodiments of this other aspect of the present disclosure are also described based on claims 11 to 17, considered alone or in any combination.

According to another aspect of the present disclosure, a controller for tracking an elongated device in an anatomy includes a memory that stores instructions; and a processor that executes the instructions. When executed by the processor, the instructions cause the system to: receive images including an elongated device; receive encoding data corresponding to driving of the elongated device; determine positions of the elongated device; determine when at least a portion of the elongated device is outside of a field of view in the images or likely to leave the field of view in the images; determine whether to create extended image data to show positions of the elongated device outside of the field of view in the images; determine current or predicted locations of the elongated device outside of the field of view in the images based on the pixel data and the encoding data; and extend the images to show the locations of the elongated device outside of the field of view in the images in extended images.

A further embodiment of this other aspect of the present disclosure is also described based on claim.

### BRIEF DESCRIPTION OF THE DRAWINGS

The example embodiments are best understood from the following detailed description when read with the accompanying drawing Figures. It is emphasized that the various features are not necessarily drawn to scale. In fact, the dimensions may be arbitrarily increased or decreased for clarity of discussion. Wherever applicable and practical, like reference numerals refer to like elements.
FIG. 1 illustrates a system for device tracking and monitoring outside imaging field of view, in accordance with a representative embodiment.
FIG. 2 illustrates a method for device tracking and monitoring outside imaging field of view, in accordance with a representative embodiment.
FIG. 3 illustrates a visualization of a coaxial guidewire controlled using a robot for device tracking and monitoring outside imaging field of view, in accordance with a representative embodiment.
FIG. 4 illustrates a system overview for device tracking and monitoring outside imaging field of view, in accordance with a representative embodiment.
FIG. 5 illustrates a visualization of a device when inside an imaging field of view, and another visualization of the device when outside the imaging field of view, in accordance with a representative embodiment.
FIG. 6 illustrates a zoom function for device tracking and monitoring outside imaging field of view, in accordance with a representative embodiment.
FIG. 7 illustrates a pan function for device tracking and monitoring outside imaging field of view, in accordance with a representative embodiment.
FIG. 8 illustrates a warping for device tracking and monitoring outside imaging field of view, in accordance with a representative embodiment.
FIG. 9 illustrates a computer system, on which a method for device tracking and monitoring outside imaging field of view is implemented, in accordance with another representative embodiment.

### DETAILED DESCRIPTION

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below.

As described herein, robot intelligence and X-ray imaging may be combined to update image acquisition and image visualization based on tracking an endovascular device outside the field of view of the X-ray system. The guidewire tip outside the field of view may be tracked by aggregating information in X-ray images and robotic encoders so as to provide an overlay for the guidewire tip on an extended image. The extended image may be obtained by combined current and past X-ray images. The generation of the extended image allows for observing the position of coaxial endovascular devices without moving the X-ray system or acquiring new X-ray images, hence improving safety and simplifying workflows.

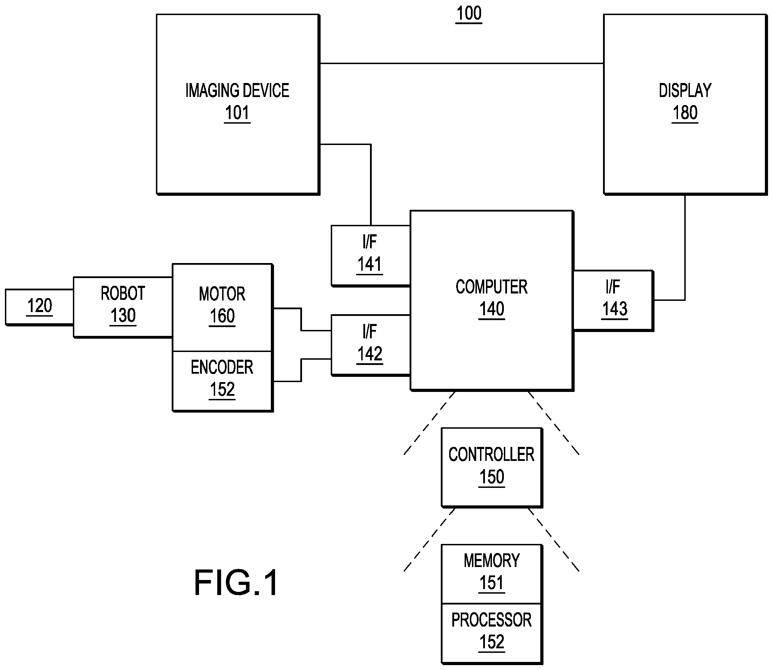

FIG. 1 illustrates a system 100 for device tracking and monitoring outside imaging field of view, in accordance with a representative embodiment.

The system 100 in FIG. 1 is a system for device tracking and monitoring outside imaging field of view and includes components that may be provided together or that may be distributed. The system 100 includes an imaging device 101, an elongated device 120, a robot 130, a computer 140, a motor 160 and a display 180.

The imaging device 101 may comprise an X-ray machine or X-ray system. The X-ray machine or X-ray system may produce 2-dimensional X-ray images. The X-ray machine or X-ray system may be configured to generate images of the elongated device 120 as 2-dimensional X-ray images.

The elongated device 120 may comprise an endovascular device. For example, the elongated device 120 may comprise a catheter with a guidewire coaxially placed inside to be advanced from and retracted to the front of the catheter. The elongated device 120 may include a distal end which advances first from the front of the catheter and a proximal end closes to the robot 130. The guidewire may not be visible in X-ray imaging when either within the catheter or when outside the field of view of the X-ray machine or X-ray system. The elongated device 120 is driven by the robot 130 under the power of the motor 160 and under the control of the computer 140.

The robot 130 may comprise a controllable device powered by the motor 160 and movable in one or more degrees of freedom. The robot 130 is configured to drive the elongated device 120 at least forward and backwards as one degree of freedom. The robot 130 may also be configured to rotate the elongated device 120 as a second degree of freedom.

The motor 160 may comprise an electric motor, a linear motor, a precision stepper motor, or a servo motor ('servo'). In some embodiments, the robot 130 and the motor 160 may be comprise an integrated unit references as either or both of a robot and/or a motor. The motor 160 is configured to drive the robot 130 to drive the elongated device 120 in the one or more degrees of freedom.
The motor 160 is also provided with an encoder 165 that tracks and encodes movement of the robot 130 and thus the elongated device 120 in the one or more degrees of freedom. The encoder 165 outputs encoded data reflecting the amount of movement of the robot 130 and thus the elongated device 120 in each degree of freedom.

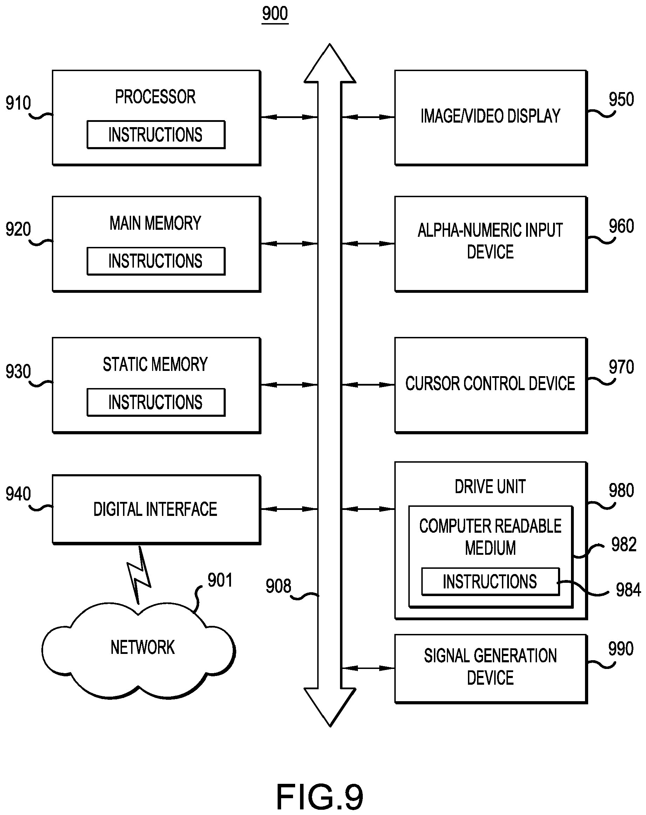

The computer 140 may comprise a workstation, a laptop, a desktop, or a specialized computer. The computer 140 includes at least a first interface 141, a second interface 142, a third interface 143, and the controller 150. A computer that can be used to implement the computer 140 is depicted in FIG. 9, though a computer 140 may include more or fewer elements than depicted in FIG. 1 or FIG. 9. The first interface 141 interfaces the computer 140 with the imaging device 101. The second interface 142 interfaces the computer 140 with the motor 160 and/or the encoder 165. The third interface 143 interfaces the computer 140 with the display 180. The first interface 141, the second interface 142 and the third interface 143 may comprise ports, adapters and/or other types of appropriate hardware configured to accept cable inputs from cables connecting to the imaging device 101, the motor 160 and/or encoder 165, and display 180.

The controller 150 includes at least a memory 151 that stores instructions and a processor 152 that executes the instructions. In some embodiments, multiple different elements of the system 100 in FIG. 1 may include a controller such as the controller 150. The controller 150 may also include interfaces, such as a fourth interface, a fifth interface, a sixth interface and a seventh interface. One or more of the interfaces of the controller 150 may include ports, disk drives, wireless antennas, or other types of receiver circuitry that connect the controller 150 to other electronic elements of the computer 140 or outside of the computer 140. One or more of the interfaces of the computer 140 or specific to the controller 150 may also include user interfaces such as buttons, keys, a mouse, a microphone, a speaker, a display separate from the display 180, or other elements that users can use to interact with the computer 140 or specifically with the controller 150 such as to enter instructions and receive output.

The controller 150 may perform some of the operations described herein directly and may implement other operations described herein indirectly. For example, the controller 150 may indirectly control operations such as by generating and transmitting content to be displayed on the display 180. For example, the controller 15 may provide display signals adapted to show on the display 180 the locations of the elongated device 120 outside of the field of view in the images. The controller 150 may directly control other operations such as logical operations performed by the processor 152 executing instructions from the memory 151 based on input received from electronic elements and/or users via the interfaces. Accordingly, the processes implemented by the controller 150 when the processor 152 executes instructions from the memory 151 may include steps not directly performed by the controller 150.

A method implemented by the system 100 under control of the controller 150 may track the elongated device 120 in an anatomy. When executed by the processor 152, instructions from the memory 151 cause the system 100 to: receive images from the imaging device 101; receive encoding data corresponding to driving of an elongated device 120 by the motor 160 from the encoder 165; determine positions of the elongated device 120; determine when at least a portion of the elongated device 120 is outside of a field of view in the images or likely to leave the field of view in the images; determine whether to create extended image data to show positions of the elongated device 120 outside of the field of view in the images on the display 180; determine at least one of current locations, past locations or predicted locations of the elongated device 120 outside of the field of view in the images based on pixel data in the images and the encoding data; and extend the images to show the locations of the elongated device 120 outside of the field of view in the images in extended images on the display 180.

For example, a software program executed by the processor 152 may include a tracking unit, an image extension unit, and other units corresponding to sub-programs of a software program described herein. The tracking unit and image extension unit are provided for tracking and visualizing the elongated device 120 from image data acquired by the imaging device 101 during a procedure. The image extension unit may be executed to create extended image data for an extended image which extends from the imaging field of view of the current image data. The extended image data may be created by the image extension unit based on other related image data. The tracking unit may be executed to receive current image data, robotic data, and the extended image data from the image extension unit, to determine the position of the elongated device 120 in the extended image when the position of a tip of the elongated device 120 is outside the imaging field of view or likely to leave the field of view in the images.

The display 180 may be local to the controller 150 or may be remotely connected to the controller 150. The display 180 may be connected to the controller 150 via a local wired interface such as an Ethernet cable or via a local wireless interface such as a Wi-Fi connection. The display 180 may be interfaced with other user input devices by which users can input instructions, including mouses, keyboards, thumbwheels and so on. The display 180 may be a monitor such as a computer monitor, a display on a mobile device, an augmented reality display, a television, an electronic whiteboard, or another screen configured to display electronic imagery. The display 180 may also include one or more input interface(s) such as those noted above that may connect to other elements or components, as well as an interactive touch screen configured to display prompts to users and collect touch input from users. The display 180 may be configured to display the locations of the elongated device 120 outside of the field of view in the images by supplementing the images with additional image data projecting the locations of the elongated device 120 outside of the field of view in the images.

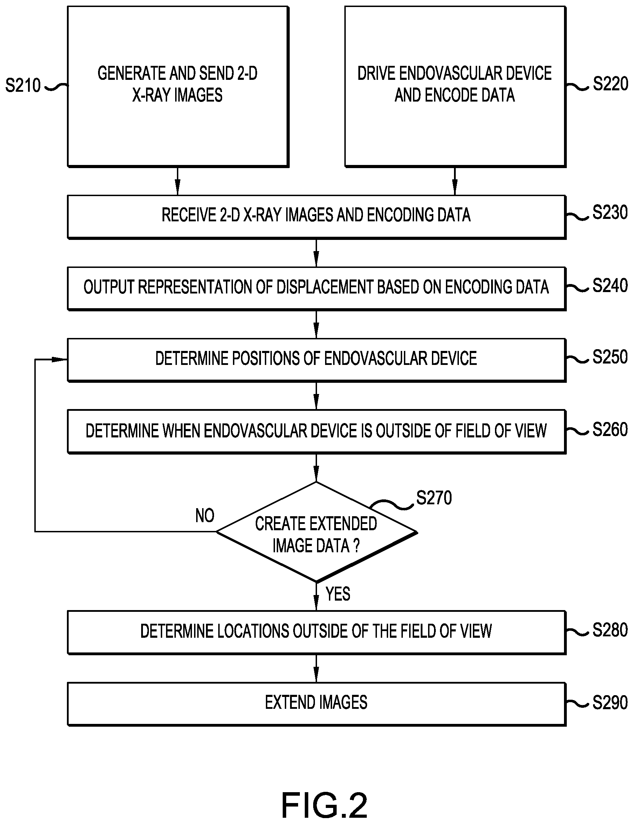

FIG. 2 illustrates a method for device tracking and monitoring outside imaging field of view, in accordance with a representative embodiment.

At S210, the method of FIG. 2 starts with generating and sending 2-dimensional X-ray images. S210 may be performed by the imaging device 101, and the 2-dimensional X-ray images may be sent to the computer 140 either directly by a connected cable or indirectly via a local area network (LAN) such as a Wi-Fi network and/or via a wide area network (WAN) such as the internet.

At S220, the method of FIG. 2 includes driving an endovascular device and encoding data. S220 may be performed by the motor 160 and the encoder 165. The encoder 165 may encode data based on the amount the elongated device 120 is being driven by the motor 160. The encoded data may include data reflective of the amount of driving in each degree of freedom. S220 may be performed simultaneously with S210 during an interventional medical procedure, and both S210 and S220 may be performed intermittently or continuously while the remainder of the method of FIG. 2 is performed.

At S230, the method of FIG. 2 includes receiving 2-dimensional X-ray images and encoding data. S230 may be performed by the computer 140 receiving X-ray images from the imaging device 101 via the first interface 141.

At S240, the method of FIG. 2 includes outputting a representation of displacement based on the encoding data. The displacement may be an estimate of movement in one or more degrees of freedom of the elongated device 120 based on a number of rotations by the motor 160, based on the amount of time the motor 160 is driving the elongated device 120, based on a speed at which the motor 160 is driving the elongated device 120, and/or based on any other factors that may be taken into account by the encoder 165. The representation of displacement may be an amount of displacement in each of the one or more degrees of freedom.

At S250, positions of an endovascular device are determined. The endovascular device may be the elongated device 120, and the positions may be determined by the controller 150. The positions may be 2-dimensional or 3-dimensional coordinates of positions of some or all of the elongated device 120 within or relative to the field of view in the X-ray images. The positions determined at S250 may include current locations, past locations, or predicted locations in the future for the elongated device 120. For example, when a tip of the elongated device 120 reenters the field of view after being outside the field of view, both the current location(s) of the tip within the field of view and the most recent past locations of the tip outside the field of view may be determined. Additionally, or alternatively, the determination of positions at S250 may occur when a tip of the elongated device 120 is likely to leave the field of view in the images, such as based on tracking the speed and directionality of the tip in past images.

At S260, the method of FIG. 2 includes determining when the endovascular device is outside of the field of view. The determination at S260 may be a determination of when at least a portion of the elongated device 120 is outside of a field of view in the images or likely to leave the field of view in the images. The likelihood of the elongated device 120 leaving the field of view may be determined based on the speed and directionality of the elongated device 120 shown in recent images.

At S270, the method of FIG. 2 includes determining whether to create extended image data. The determination at S270 is based on whether the endovascular device is outside of the field of view or likely to move outside of the field of view. The extended image data may provide an extended image, such as in a different color or shading, with a separate border or type of border, or which otherwise delineates to an operator that the extended image is an artificial projection outside the field of view and extended from the actual image within the field of view. If extended image data is not to be created (S270 = No), the method of FIG. 2 returns to S250.

If extended image data is to be created (S270 = Yes), the method of FIG. 2 includes determining locations of the elongated device outside of the field of view at S280. The determination at S280 may be a determination of at least one of current locations, past locations or predicted locations of the elongated device outside of the field of view in the images based on pixel data in the images and the encoding data. The current or predicted locations may include estimated locations of a tip of the elongated device 120. For example, the processor 152 may provide display signals adapted to show on the display 180 the locations of the elongated device 120 outside of the field of view in the images.

At S290, the method of FIG. 2 includes extending the X-ray images to project locations of the elongated device outside of the field of view. Imagery of the tip of the elongated device 120 may be overlaid on the extended images at the locations determined at S280 based on the pixel data and the encoding data. Extending the X-ray images at S290 may be based on at least one of current image data of current images and past image data of past images. The images may be extended at S290 by at least one of warping, mosaicking, or stitching at least one image. A representation of a displacement of the elongated device 120 may be output based on the encoding data, and the representation of the displacement of the elongated device 120 may be generated based on the warping, mosaicking or stitching the at least one image.

As set forth above, the method of FIG. 2 may include tracking a tip of an elongated device 120 both when the tip is within the field of view of the images, as well as when the tip leaves or enters the field of view of the images. The extended image generated to show the current, past or projected location of the tip may assist an operator to understand the context and potential danger presented by any part of the elongated device 120 being outside of the field of view. In some embodiments, the system 100 may generate alarms based on movement of the elongated device 120, such as based on current, past or future location, velocity, or any other metric available to the system 100.

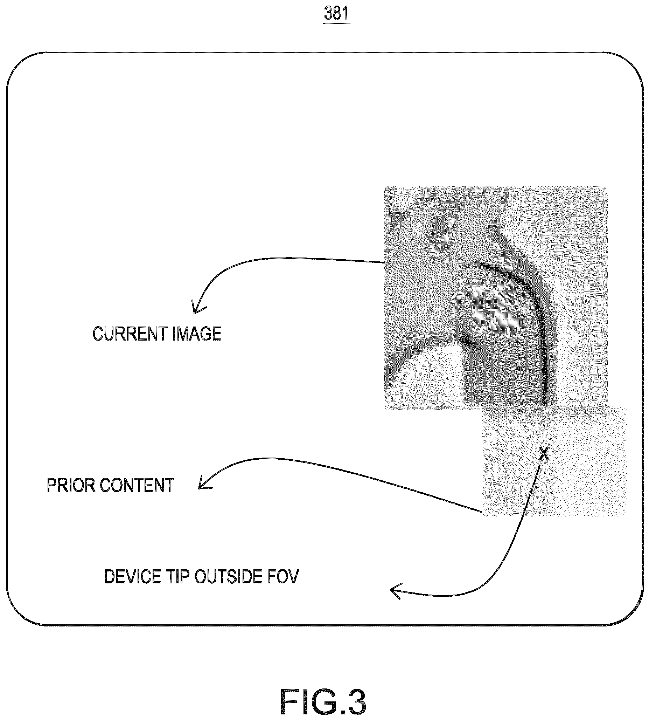

FIG. 3 illustrates a visualization of a coaxial guidewire controlled using a robot for device tracking and monitoring outside imaging field of view, in accordance with a representative embodiment. The visualization in FIG. 3 is provided on a user interface 381, such as on the display 180 in FIG. 1. The current image shows an elongated device in descending aorta. The extended X-ray image is provided beneath the current image as prior context, and includes an X marking the guidewire tip outside the field of view (FOV).

FIG. 3 illustrates a visualization solution in which the current position of the guidewire tip that is tracked using the robot data is overlayed on previously acquired X-ray images. The coaxial guidewire is controlled using the robot even when the coaxial guidewire is outside the field of view. The guidewire tip is shown with a cross in FIG. 3.

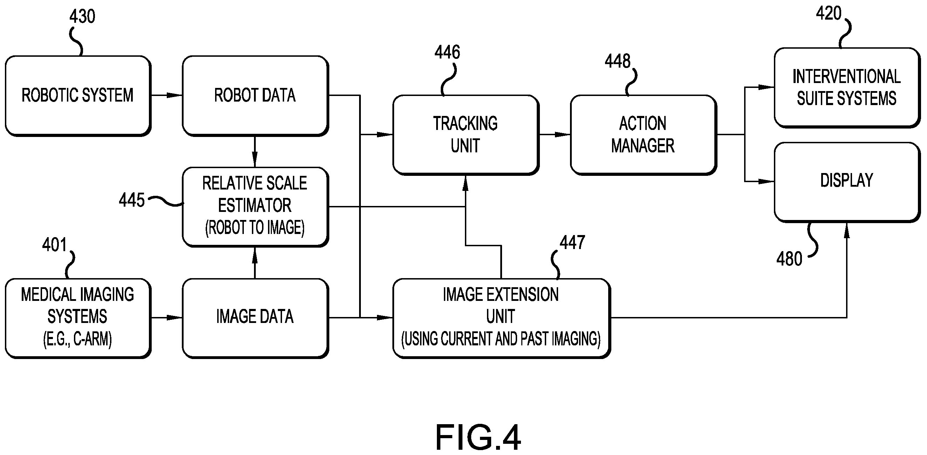

FIG. 4 illustrates a system overview for device tracking and monitoring outside imaging field of view, in accordance with a representative embodiment.

In FIG. 4, a processing unit implemented by the controller 150 in FIG. 1 uses the robot data and image data, computes the position of the elongated device 120 outside the imaging field of view, and overlays the position of the tip of the elongated device 120 on extended view images.

The system overview in FIG. 4 includes one or more medical imaging systems 401, interventional suite 420, a robotic system 430, a relative scale estimator 445, a tracking unit 446, an image extension unit 447, an action manager 448, and a display 480.

The medical imaging systems 401 may include a C-arm X-ray system, for example. The medical imaging systems 401 outputs image data such as X-ray data.

The interventional suite 420 may be software used to process X-ray images from the medical imaging systems 401, including raw X-ray data and processed data processed via the relative scale estimator, the tracking unit 446, the image extension unit 447 and the action manager 448.

The robotic system 430 may include either or both of the robot 130 and the motor 160, along with the encoder 165 in FIG. 1. The robotic system 430 providing robotic data related to device position. The robotic system 430 outputs the robotic data.

The relative scale estimator 445, the tracking unit 446, the image extension unit 447, and the action manager 448 may be implemented by software stored in the memory 151 of the controller 150 in FIG. 1, along with one or more instances of the processor 152 of the controller 150 in FIG. 1.

The relative scale estimator 445 receives the imaging data and robotic data. The relative scale estimator 445 is configured to receive (i) current image data in an imaging field of view and (ii) robotic data, and output a scalar parameter representative of a displacement of the elongated device in the real-time image associated with robotic data. Due to foreshortening and projective geometry of X-ray, the scale may differ per pixel. The extended image data results in warping of the image for stitching, and the relative scale estimator 445 necessarily takes into account this warping to estimate the actual position.

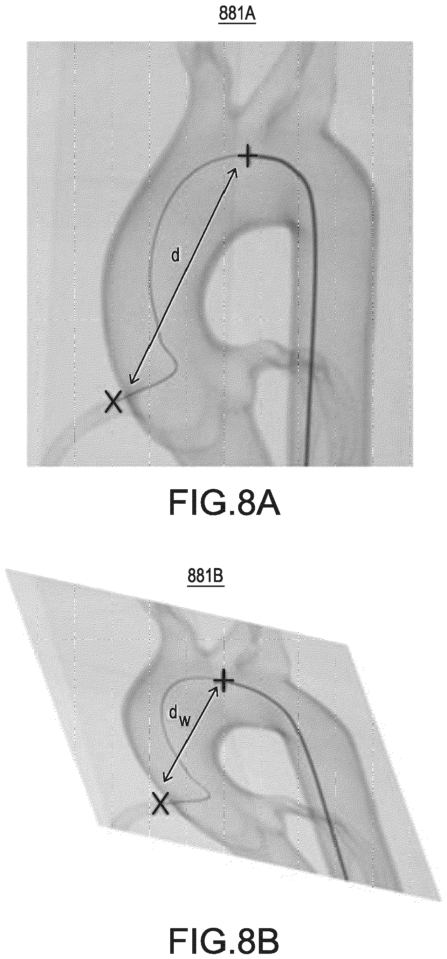

The relative scale estimator 445 computes a scalar parameter which is the ratio of robot actuation over displacement in the image coordinate. Given this scale, the controller 150 may predict that a particular robot actuation command results in what amount of displacement at the tip of the elongated device 120 in image coordinates. Due to the foreshortening and projective geometry of X-ray, this scale is different for every pixel coordinate in the image, every device type, every robot configuration, every anatomy, etc. The relative scale estimator 445 may be implemented using a trained artificial intelligence model or a lookup table, each of which are explained later. It should be noted that images will be warped for stitching and creating the extended view, hence the computed scale factor will change as the result of image warping. Warping is addressed in the description of FIG. 8, including how to update the scale factors using interpolation.

The tracking unit 446 and the image extension unit 447 provide for tracking and visualizing, from image data acquired by the medical imaging systems 401, an interventional device controlled by the robotic system 430 during a procedure. The tracking unit 446 uses extended images to compute the position of the tip of the elongated device 120 outside or at the border of the imaging field of view. The tracking unit 446 uses the scalar parameter from the relative scale estimator 445 to determine the position of the elongated device.

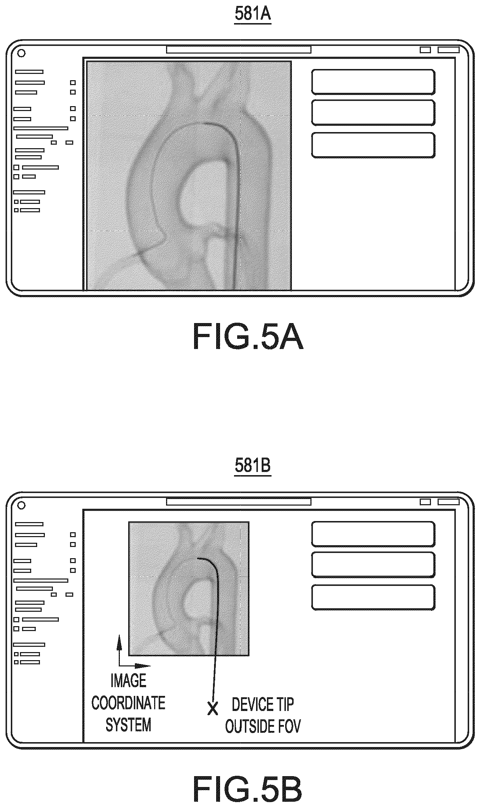

The tracking unit 446 computes displacement of tip of the elongated device 120 when the tip is outside the imaging field of view. The tracking unit 446 receives the extended image canvas, the dynamic scale factor, the robot data, and computes the position of the tip with respect to the image coordinate system as shown in FIG. 5. The tracking unit 446 constantly computes the position of the elongated device 120 in the general workflow by combining robot and image data. When the elongated device 120 leaves the field of view, the tracking unit 446 will notify the action manager 448, which triggers updates on the display 480 or the interventional suite 420.

The image extension unit 447 is arranged to create an extended image data which extends from the imaging field of view of the current image data. The extended image data is created based on other related image data. The tracking unit 446 is arranged to receive (i) current image data and (ii) robotic data, and extended image data from the image extension unit 447, to determine the position of the guidewire tip in the extended image when the position of the guidewire tip is outside the imaging field of view. The extended image data from the image extension unit 447 uses the most recent and prior images. Prior imaging may include both previous X-ray images obtained during the same procedure from the same patient or 2-dimensional images synthesized from prior 3-dimensional imaging, such as digitally reconstructed radiographs.

The image extension unit 447 creates an extended graphical interface using the most recent and prior images as shown in FIG. 3. The prior imaging includes both previous X-ray images obtained during the same procedure from the same patient or 2-dimensional images synthesized from prior 3-dimensional imaging such as digitally reconstructed radiographs.

Once the position of the tip of the elongated device 120 outside the field of view is determined by the tracking unit 446, the action manager 448 will either update the display 480 to display the guidewire tip outside the imaging field of view or trigger another event, such as changing the imaging settings to capture the guidewire tip within a new imaging field of view or controlling the motion of the robotically controlled device accordingly. Changes of the imaging settings may include zooming out or panning, for example. Depending on the workflow, phase, or user preferences, the action manager 448 unit will either update the display to visualize the position of the device tip as shown in FIG. 3 or will trigger event in the interventional suite 420, such as changing the imaging settings to capture the device tip within the imaging field of view or controlling the motion of the robotically controlled device accordingly.

Using the system in the system overview of FIG. 4, the guidewire tip is tracked outside the field of view by aggregating the information in X-ray images and the robotic encoders. The system provides an overlay for the guidewire tip on an extended image on the display 480. The extended image is obtained by combined current and past X-ray images.

FIG. 5 illustrates a visualization of a device when inside an imaging field of view, and another visualization of the device when outside the imaging field of view, in accordance with a representative embodiment.

The visualizations in FIG. 5 are shown as a sequence of user interfaces such as displays on the display 180. The sequence includes a first user interface 581A for the visualization of the device when inside of the imaging field of view, and the second user interface 5 8 1B for the visualization of the device when outside of the imaging field of view.

In FIG. 5, the live X-ray images are displayed in the graphical interface when the elongated device 120 is in the field-of-view and imaging is active on the first user interface 581A. When the elongated device 120 leaves the field-of-view and the image acquisition is paused, the controller 150 computes the position of the elongated device 120 with respect to the image coordinate system for the second user interface 581B.

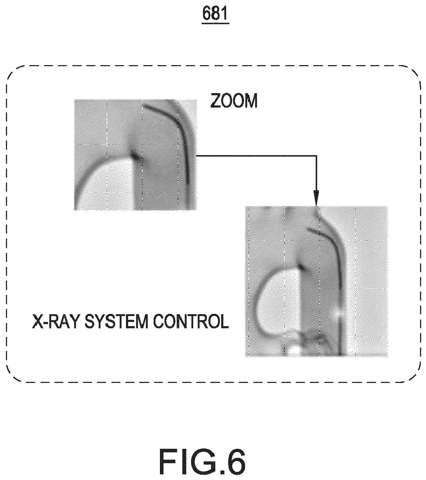

FIG. 6 illustrates a zoom function for device tracking and monitoring outside imaging field of view, in accordance with a representative embodiment.

The zoom function in FIG. 6 is provided on a user interface 681, such as on the display 180 in FIG. 1. In FIG. 6, an elongated device in a shoulder and an arm on the upper left is zoomed out on the lower right, such as by controlling the imaging device 101 in FIG. 1 via the controller 150 in FIG. 1.

An embodiment that controls the zoom of the system depending on the state of the device tip when it is outside the field of view. For example, if the tip of hidden device leaves the field of view: 1) zoom out, 2) reacquire X-ray image(s), 3) overlay tip location on the new image(s).

As shown in FIG. 6, the imaging acquisition parameters associated with changing scale can be triggered and adjusted based on the device's position outside the current imaging frustum.

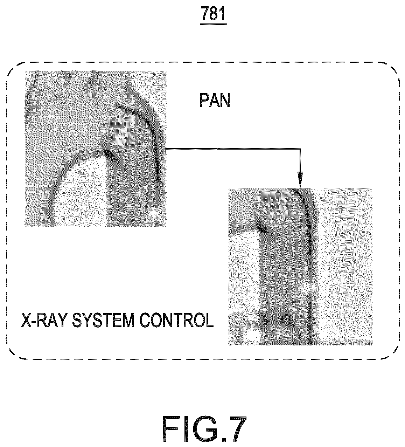

FIG. 7 illustrates a pan function for device tracking and monitoring outside imaging field of view, in accordance with a representative embodiment.

The pan function in FIG. 7 is provided on a user interface 781, such as on the display 180 in FIG. 1. In FIG. 7, an elongated device in a shoulder and an arm on the upper left is panned to the left on the lower right, such as by controlling the imaging device 101 in FIG. 1 via the controller 150 in FIG. 1.

An embodiment that controls the pan of the system 100 depending on the state of the tip of the elongated device 120 when outside the field of view is shown in FIG. 7. For example, if the tip of a hidden guidewire as the elongated device 120 leaves the field of view, the imaging view may be panned in the direction of the location of the top, 2) X-ray image(s) may be reacquired, and 3) tip location may be overlayed on the new image(s). For example, when the device reaches the borders of the image, a C-arm system may pan so that the guidewire device remains visible.

FIG. 8 illustrates a warping for device tracking and monitoring outside imaging field of view, in accordance with a representative embodiment.

As shown in FIG. 8, the relative scale in the original (a) and warped (b) images are different d^dw. FIG. 8 illustrates warping to adapt relative scale to outside the field of view. Warping in FIG. 8 is shown via two user interfaces including the first user interface 881A and the second user interface 881B. The warping reflects complexity of estimating depth of positions of an elongated device in anatomy when captured in a 2-dimensional image from a 2-dimensional imaging system such as a 2-dimensional X-ray system. The 2-dimensional image in the first user interface 881A shows a distance d between a guidewire tip and a designated position. When the elongated device is not perpendicular to the imager of the 2-dimensional imaging system, the controller 150 uses a warping technique to transform the positions on the first user interface 881A to the warped positions on the second user interface 881B. Warping the images for stitching will require the relative scales to update to match the scale in the warped images (FIG. 8). This embodiment proposes a method to update the relative scales based on the warping of images. To do this, corresponding key-points are identified in the original and warped images. The key-points could be hand-crafted features (e.g., SIFT) or context-specific landmarks (e.g., the tip of endovascular device). The cross-ratio or the distance of change in landmarks is considered as the update factor (u = dw / d). Finally, for each point in the warped image, the new scale is obtained by multiplying the original scale s with the update factor: su = s * u.

In some other embodiments, pause may be implemented by the controller 150. For example, the controller 150 may pause actuation of the elongated device 120 when the tip of the elongated device 120 is exiting the image boundary.

In some embodiments, gain control may be implemented by the controller 150. For example, the controller 150 may adjusts gain for the robot 130 actuating the elongated device 120 depending on if the elongated device 120 is entering or exiting the image boundary. For example, the gain may be reduced when the elongated device 120 leaves the field of view to increase safety.

In some embodiments, digitally reconstructed radiographs may be created by the controller 150. When the elongated device 120 leaves the field of view, digitally reconstructed radiographs may be generated from prior 3-dimensional imaging to visualize the tip of the elongated device 120 outside the field of view instead of stitching different images.

In some embodiments, a relative scale estimator may be implemented using a trained artificial intelligence model. The relative calibration scales may be learned by an artificial intelligence model. Neural networks with convolutional, fully connected, and recurrent layers may be used to learn the scale value at the tip of the elongated device 120 given imaging and robotic data. The neural network may also use the segmentation of different devices and anatomies as input. The ground-truth labels may be generated retrospectively from prior robotic navigations or developed in synthetic environments.

In some embodiments, a relative scale estimator may be implemented using a lookup table. For example, the relative scale factors may be stored in a lookup table for those locations in the image that the catheter tip traveled through while the catheter device was advancing in the vasculature. Then, when the guidewire is retracted inside the catheter, the corresponding scales may be approximated by assigning the closest pixel with a scale label from the lookup table.

FIG. 9 illustrates a computer system, on which a method for device tracking and monitoring outside imaging field of view is implemented, in accordance with another representative embodiment.

Referring to FIG.9, the computer system 900 includes a set of software instructions that can be executed to cause the computer system 900 to perform any of the methods or computer-based functions disclosed herein. The computer system 900 may operate as a standalone device or may be connected, for example, using a network 901, to other computer systems or peripheral devices. In embodiments, a computer system 900 performs logical processing based on digital signals received via an analog-to-digital converter.

In a networked deployment, the computer system 900 operates in the capacity of a server or as a client user computer in a server-client user network environment, or as a peer computer system in a peer-to-peer (or distributed) network environment. The computer system 900 can also be implemented as or incorporated into various devices, such as a workstation that includes a controller, a stationary computer, a mobile computer, a personal computer (PC), a laptop computer, a tablet computer, or any other machine capable of executing a set of software instructions (sequential or otherwise) that specify actions to be taken by that machine. The computer system 900 can be incorporated as or in a device that in turn is in an integrated system that includes additional devices. In an embodiment, the computer system 900 can be implemented using electronic devices that provide voice, video or data communication. Further, while the computer system 900 is illustrated in the singular, the term "system" shall also be taken to include any collection of systems or sub-systems that individually or jointly execute a set, or multiple sets, of software instructions to perform one or more computer functions.

As illustrated in FIG. 9, the computer system 900 includes a processor 910. The processor 910 may be considered a representative example of a processor of a controller and executes instructions to implement some or all aspects of methods and processes described herein. The processor 910 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 910 is an article of manufacture and/or a machine component. The processor 910 is configured to execute software instructions to perform functions as described in the various embodiments herein. The processor 910 may be a general-purpose processor or may be part of an application specific integrated circuit (ASIC). The processor 910 may also be a microprocessor, a microcomputer, a processor chip, a controller, a microcontroller, a digital signal processor (DSP), a state machine, or a programmable logic device. The processor 910 may also be a logical circuit, including a programmable gate array (PGA), such as a field programmable gate array (FPGA), or another type of circuit that includes discrete gate and/or transistor logic. The processor 910 may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The computer system 900 further includes a main memory 920 and a static memory 930, where memories in the computer system 900 communicate with each other and the processor 910 via a bus 908. Either or both of the main memory 920 and the static memory 930 may be considered representative examples of a memory of a controller, and store instructions used to implement some or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The main memory 920 and the static memory 930 are articles of manufacture and/or machine components. The main memory 920 and the static memory 930 are computer-readable mediums from which data and executable software instructions can be read by a computer (e.g., the processor 910). Each of the main memory 920 and the static memory 930 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, Blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system 900 further includes a video display unit 950, such as a liquid crystal display (LCD), an organic light emitting diode (OLED), a flat panel display, a solid-state display, or a cathode ray tube (CRT), for example. Additionally, the computer system 900 includes an input device 960, such as a keyboard/virtual keyboard or touch-sensitive input screen or speech input with speech recognition, and a cursor control device 970, such as a mouse or touch-sensitive input screen or pad. The computer system 900 also optionally includes a disk drive unit 980, a signal generation device 990, such as a speaker or remote control, and/or a network interface device 940.

In an embodiment, as depicted in FIG. 9, the disk drive unit 980 includes a computer-readable medium 982 in which one or more sets of software instructions 984 (software) are embedded. The sets of software instructions 984 are read from the computer-readable medium 982 to be executed by the processor 910. Further, the software instructions 984, when executed by the processor 910, perform one or more steps of the methods and processes as described herein. In an embodiment, the software instructions 984 reside all or in part within the main memory 920, the static memory 930 and/or the processor 910 during execution by the computer system 900. Further, the computer-readable medium 982 may include software instructions 984 or receive and execute software instructions 984 responsive to a propagated signal, so that a device connected to a network 901 communicates voice, video or data over the network 901. The software instructions 984 may be transmitted or received over the network 901 via the network interface device 940.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Accordingly, device tracking and monitoring outside imaging field of view enables combination of robot intelligence and X-ray imaging to update image acquisition or image visualization based on tracking an endovascular device outside the field of view in the X-ray imaging. Guidewire tips outside the field of view may be tracked by aggregating information in X-ray images and robotic encoders so as to provide an overlay for the guidewire tip on an extended image. Positions of coaxial endovascular devices may be observed without moving the X-ray system or acquiring new X-ray images, hence improving safety and simplifying workflows. As a result, procedure safety is increased since the teachings herein allow tracking and visualization of the elongated device even outside the field of view; hence dangerous maneuvers and motions can be viewed and mitigated. Additionally, X-ray radiation is reduced as additional X-ray imaging is rendered unnecessary in some circumstances for tracking and visualizing the elongated device. Moreover, the workflow is improved as unnecessary changes to the imaging setup are eliminated.

Although device tracking and monitoring outside imaging field of view has been described with reference to several exemplary embodiments, it is understood that the words that have been used are words of description and illustration, rather than words of limitation. Changes may be made within the purview of the appended claims, as presently stated and as amended, without departing from the scope and spirit of device tracking and monitoring outside imaging field of view in its aspects. Although device tracking and monitoring outside imaging field of view has been described with reference to particular means, materials and embodiments, device tracking and monitoring outside imaging field of view is not intended to be limited to the particulars disclosed; rather device tracking and monitoring outside imaging field of view extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the Figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. § 1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A system for tracking an elongated device driven by at least one motor in an anatomy, comprising:
a controller configured to:
receive images;
receive encoding data corresponding to driving of the elongated device by the motor;
determine positions of the elongated device;
determine when at least a portion of the elongated device is outside of a field of view in the images or likely to leave the field of view in the images;
determine whether to create extended image data to show positions of the elongated device outside of the field of view in the images;
determine at least one of current locations, past locations or predicted locations of the elongated device outside of the field of view in the images based on pixel data in the images and the encoding data; and
extend the images to show the locations of the elongated device outside of the field of view in the images in extended images.

2. The system of claim 1, further comprising:
an X-ray machine that generates the images of the elongated device as 2-dimensional X-ray images, wherein the elongated device comprises an endovascular device.

3. The system of claim 1, wherein the controller is further configured to:
provide display signals adapted to show on a display the locations of the elongated device outside of the field of view in the images by supplementing the images with additional image data projecting the locations of the elongated device outside of the field of view in the images.

4. The system of claim 1, wherein the current or predicted locations include estimated locations of a tip of the elongated device, and imagery of the tip of the elongated device is overlaid on the extended images at the locations determined based on the pixel data and the encoding data.

5. The system of claim 1, wherein the controller is further configured to:
update acquisition of the images based on the encoding data and the pixel data to return the elongated device into the field of view in the images.

6. The system of claim 1, wherein the system determines to create the extended image data based on determining that the elongated device is outside of a field of view in the images, and wherein the elongated device comprises an endovascular device.

7. The system of claim 1, wherein the controller is further configured to:
receive current image data and current encoding data and generate and output based on the encoding data a representation of a displacement of the elongated device in the current image data.

8. The system of claim 7, wherein the controller is further configured to:
extend the images by at least one of warping, mosaicking, or stitching at least one image, wherein the representation of a displacement is generated based on the warping, mosaicking or stitching the at least one image.

9. The system of claim 1, wherein the controller is further configured to:
extend the images based on at least one of current image data of current images and past image data of past images.

10. A method for tracking an elongated device in an anatomy, comprising:
receiving, at a system comprising a memory that stores instructions and a processor that executes the instructions, images of an elongated device;
receiving encoding data corresponding to driving of the elongated device;
determining positions of the elongated device;
determining when at least a portion the elongated device is outside of a field of view in the images or likely to leave the field of view in the images;
determining whether to create extended image data to show positions of the elongated device outside of the field of view in the images;
determining current or predicted locations of the elongated device outside of the field of view in the images based on pixel data in the images and the encoding data; and
extending the images to show the locations of the elongated device outside of the field of view in the images in extended images.

11. The method of claim 10, further comprising:
displaying, on a display, the locations of the elongated device outside of the field of view in the images by supplementing the images with additional image data projecting the locations of the elongated device outside of the field of view in the images.

12. The method of claim 10, wherein the current or predicted locations include estimated locations of a tip of the elongated device, and imagery of the tip of the elongated device is overlaid on the extended images at the locations determined based on the pixel data and the encoding data.

13. The method of claim 10, further comprising:
receiving current image data and current encoding data and generating and outputting based on the encoding data a representation of a displacement of the elongated device in the current image data.

14. The method of claim 13, further comprising:
extending the images by at least one of warping, mosaicking, or stitching at least one image, wherein the representation of a displacement is generated based on the warping, mosaicking or stitching the at least one image.

15. A controller for tracking an elongated device in an anatomy, comprising:
a memory that stores instructions; and
a processor that executes the instructions, wherein, when executed by the processor, the instructions cause a system to:
receive images including an elongated device;
receive encoding data corresponding to driving of the elongated device;
determine positions of the elongated device;
determine when at least a portion of the elongated device is outside of a field of view in the images or likely to leave the field of view in the images;
determine whether to create extended image data to show positions of the elongated device outside of the field of view in the images;
determine current or predicted locations of the elongated device outside of the field of view in the images based on pixel data in the images and the encoding data; and
extend the images to show the locations of the elongated device outside of the field of view in the images in extended images.
